# EUROPEAN PATENT SPECIFICATION

(11) **EP 2 098 506 B1**
(45) Date of publication and mention of the grant of the patent: **28.07.2010**
(21) Application number: 08003291.5
(22) Date of filing: 22.02.2008
(51) Int. Cl.: C07D 201/06

(54) **Production of lactams by ammoximation of cyclic ketones**
Herstellung von Lactamen durch Ammoximierung zyklischer Ketone
Production de lactames par la production d'oximes de cycloalkanones

(43) Date of publication of application: 09.09.2009
(73) Proprietor: Uhde Inventa-Fischer GmbH, 13509 Berlin (DE)
(72) Inventor: Hölderich, Wolfgang F., 67227 Frankenthal (DE); Anilkumar, Mettu, 52074 Aachen (DE); Schaller, Rainer, 86637 Wertingen (DE)
(74) Representative: Pfenning, Meinig & Partner GbR

(56) References cited:
- RAJA, R., SANKAR, G., THOMAS, J. M.;: "Bifunctional Molecular Sieve Catalysts for the Benign Ammoximation of Cyclohexanone: One-Step, Solvent-Free Production of Oxime and Caprolactam with a Mixture of air and Ammonia" J. AM. CHEM. SOC., vol. 123, 2001, pages 8153-8154, XP002488749

## Description

Present invention describes the direct catalytic liquid phase synthesis of caprolactam from cyclohexanone, hydrogen peroxide and ammonia. The invention can be carried out by using mesoporous materials amongst the MMS family (mesoporous molecular sieves) as catalysts, such as Ti-MCM-41, Al-MCM-41 and Ti-SBA-15 materials at temperatures between 20 and 120 °C.

In a conventional method, caprolactam is produced by Beckmann rearrangement, where cyclohexanone oxime was transformed into caprolactam in the presence of concentrated sulfuric acid or phosphoric acid as catalysts, followed by neutralization with liquid ammonia. The cyclohexanone oxime needed thereby, is obtained by the oximation of cyclohexanone either via hydroxylamine in the presence of sulfuric acid or via H₂O₂/NH₃ mixture over TS-1 zeolite. TS-1 zeolite means titanium-silica zeolite with MFI-structure (pentasil zeolite structure).

These methods have a number of disadvantages, such as the separation of caprolactam from the product mixture, undesirable large quantities of ammonium sulfate or ammonium phosphate formation as well as equipment corrosion.

To overcome these problems, there has been proposed a method of cyclohexanone oxime rearrangement reaction in the presence of heterogeneous catalysts, such as solid acid catalysts and zeolite catalysts which showed excellent activity and selectivity to caprolactam (EP 1 193 251; US 6,531,595; Catalysis Reviews 43(4), 381-441 (2001); Journal of Catalysis 194, 122-129 (2000).

These reactions are carried out in vapor phase or liquid phase reaction conditions.

Cyclohexanone oxime is produced by the EniChem process by ammoximation of cyclohexanone with hydrogen peroxide and ammonia in presence of TS-1 catalyst (US 4,794,198; EP 0 267 362; ACS Symposium Series (1996), 626 (Green Chemistry), 33-48).

In this process, reactions are operated under mild liquid phase conditions and high yields of cyclohexanone oxime using TS-1 catalyst are obtained.

Gas phase ammoximation of cyclohexanone was first reported by J.N. Armor et al. in 1979. In this reaction cyclohexanone vapor reacts with air and ammonia in the presence of solid catalysts e.g. (1) amorphous silica/alumina gel (US 4,281,194) or (2) alumina silicate catalysts (US 4,163,756; Journal of Catalysis 70, 72 (1981); Journal of Catalysis 70, 91 (1981); Journal of Catalysis 72, 66 (1981).

The main objective of the present invention is therefore the synthesis of caprolactam by one step synthesis from cyclohexanone and a mixture of ammonia and hydrogen peroxide in presence of mesoporous and/or zeolitic materials as catalysts under liquid phase reaction conditions.

This objective is solved by the features of patent claim 1. The dependent claims describe advantageous embodiments.

According to present invention, a method of producing a cyclic lactam of the generic formula I 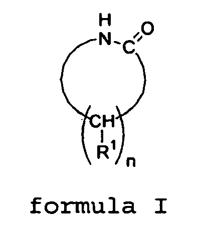 is provided, starting from cyclic ketones of the generic formula II 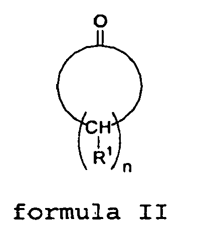 wherein in both formulae I and II
n is 2 to 20, and
R¹ independently is selected from the group consisting of hydrogen, linear or branched C₁-C₁₂-alkyl, linear or branched alkenyl with up to 12 carbon atoms, linear or branched alkinyl with up to 12 carbon atoms, aromatic residues with 6 to 18 carbon atoms, aralphatic residues with 7 to 20 carbon atoms and/or residues which contain heteroatoms,
by reacting the cyclic ketone in presence of at least one heterogeneous catalyst system with ammonia and at least one oxidant by conducting the reaction in one step and in liquid phase, by using an aqueous solution of hydrogen peroxide as oxidant.

In such, caprolactam is produced by the cyclohexanone ammoximation method, in which cyclohexanone is reacted with hydrogen peroxide and ammonia preferably in the presence of MMS family materials as catalysts under liquid phase reaction conditions.

It is preferred, if the heterogeneous catalyst system contains at least one material which includes transition metals and/or IIA and/or IIIA and/or IVA main group elements. In a further advantageous embodiment, the catalyst system can contain a material with mesoporous structure and/or zeolitic structure and/or a material selected from the group of the mesoporous molecular sieves. Especially preferred examples for the mesoporous molecular sieves are selected from the group consisting of materials with MCM-41-structure, MCM-48-structure, HMS-structure and/or SBA-15-structure.

Examples of the mesoporous materials are Ti-MCM-41, Al-MCM-41 and Ti-SBA-15 catalysts (US 5,712,402;
US 6,770,258).

Among the mesoporous materials, silica substituted materials with hetero atoms have been showing considerable activity, due to easily accessible active sites within their framework.

In the present invention, catalysts, such as MFI structure zeolites, TS-1, SnS-1, Ti-Sn-S-1, and TiO₂ on SiO₂ support, were also used and further more for the comparison study with MMS family catalysts.

Ti-MCM-41 material was prepared by following hydrothermal method (Microporous and Mesoporous Materials (1998), 21(1-3), 67-74). In a teflon vessel, silica aerosil and water were mixed to form a homogeneous solution. Decyltrimethyl ammoniumbromide was added as structure agent to this solution and stirred for 1 h. A colorless solution formed. To this colorless solution, the required amount of 40 wt% TEAOH (tetra-ethylammoniumhydroxide) was added under vigorous stirring. The resulting solution was stirred for 1 h at room temperature. Then, [(NH₄)₃[Ti(O₂)F₅] was added to the mixture and stirred for another 1 h at room temperature. A yellow solution formed. To this solution, the required amount of silica aerosil was added and stirred for 5 h at room temperature, after which a yellow color gel formed. This gel was transferred into an autoclave and heated at 100 °C for 7 days to complete crystallization. After crystallization, the product was filtered off, washed with deionized water and dried at 100 °C for 12 h. A finally dried sample was calcined at 500 °C for 8 h.

In case of the Ti-MCM-41 sample, a titanium source and in the case of Al-MCM-41 catalysts, an alumina source was used (Microporous Material 5 (1995), 211). Al-MCM-41 was prepared according to a slightly altered method of the reference mentioned above. In a 250 ml polyethylene flask, 10,5 g tetraethylammonium hydroxide (TEAOH, 40 wt% aqueous), 0,21 g NaAlO₂ and 50 g H₂O were mixed together and stirred at room temperature for 1 h, followed by the addition of 10 g tetradecyltrimethylammonium bromide. The resulting mixture was stirred for 4 h. Then, 15,23 g Ludox-HS-40 (means a silicasol, Tradename of DuPont, Wilmington) were added dropwise over a period of 1 h, following by vigorous stirring at ambient temperature for 4 h. The crystallization.took place at 105 ° C for 6 days. After the third and fifth day, the pH was adjusted to 10,2 using CH₃COOH (10 wt % aqueous). After crystallization, the solid was dried at 120 °C overnight, followed by calcination under static air at 540 °C for 6 h.

Ti-SBA-15 was prepared by using a SBA-15 (it is a name of a mesoporous material) template by the hydrothermal synthesis method. The hydrothermal synthesis of almost Al-free Ti-SBA-15 Si/Ti 42.45 was carried out. In a typical synthesis, 10 g of triblock polymer (polyalkylene oxid triblock copolymer) was dispersed in 75 g of double-distilled water at 40 °C. The resultant solution was stirred with 300 g of 2 M HCl solution containing the requisite amount of TPOT (tetraisopropylorthotitanate) to obtain a homogeneous solution. Finally, 21,25 g of TEOS (tetraethoxysilane) was added to the above mixture at 40 °C under vigorous stirring for 2 h. This solution was aged without stirring at 40 °C for 12 h and 80 °C for 24 h. The solid products were recovered, washed, and air-dried at room temperature. The solid was calcined by slowly increasing the temperature from room temperature to 500 °C over 8 h after which the temperature was maintained at 500 °C for a further 6 h. Examples of microporous materials TS-1, titanostannosilicalites, and stannosilicates were prepared according to the method (US 4,410,501, US 5,780,654; J. CHEM. SOC., CHEM. COMMUN., 1933-1994 (1994).

However, it is also possible, if the catalyst contains Al, Si, Ti, Mn, Sn, V, Mo, Cu, Co, Fe, B, Ga, Ge, Nb and/or Mg as active centres of the catalyst system.

The starting material of this invention, i.e. the cyclic ketone according to formula II, e.g. cyclohexanone, was obtained by different methods, for example cyclohexane oxidation, cyclohexene hydration and hydrogenation or dehydrogenation of phenol.

Hydrogen peroxide is usually produced by anthraquinone process and is commercially available in the form of an aqueous solution having a concentration of 10 % by weight to 70 % by weight. In the present invention, preferably 30 wt% hydrogen peroxide in the form of aqueous solution was used. The relative molecular amount of the hydrogen peroxide to be used is preferably in the range between 0,5 mol and about 3 mol. It is more preferably in the range between 0,5 mol and 1,5 mol, based on 1 mol of the cyclohexanone.

Ammonia used in the invention is either in gas form or in aqueous solution. In this invention 28 wt% ammonia solution is used. But also more diluted or more concentrated ammonia solutions can be applied as well as gaseous pure ammonia.

The relative amount of ammonia used in the invention is in between 0,5 and 3 mol, preferably 1 mol or more, and more preferably about 2 mol, based on 1 mol of the cyclohexanone.

Preferably, solvents are employed in the reaction to form a homogeneous mixture of reactants. Solvents which do not react or interact with the mixture of reactants and/or the catalyst under reaction conditions are especially advantageous. Useful solvents in these reactions are alcohols, such as methanol, ethanol, n-propanol, i-propanol, n-butanol, s-butanol, t-butanol and/or acetone, acetonitrile, water and/or mixtures thereof.

In this invention, 0 to 100 wt%, preferably 5 to 50 wt% of solvents based on the sum of the reactants are used. Preferably, about 10 wt% of the solvent is used.

The titled method is preferably carried out in a liquid phase batch reactor, although stirred tank reactor, loop reactor, continuous flow reactor, fluidized bed or other types of reactors can be useful, too. The reaction temperature is in the range of about room temperature to about 120 °C, and more preferably between about 60 °C to about 100 °C. Optimum temperature is around 70 to 80°C.

Although the reaction can be carried out at atmospheric pressure, reduced and elevated pressure from about 0,5 bars to about 40 bars, and more preferably from about 10 bars to about 30 bars can be applied.

At lower temperatures, the reaction kinetics is rather slow while at higher temperatures the negative effect of both parallel reactions and consecutive reactions becomes predominant (decomposition of H₂O to H₂O + O₂).

The present invention is preferably carried out with magnetic stirrer speeds about 500 to 1500 rotations per minute (RPM). Optimum stirring speed for the reaction is around 900 to 1100 rpm. The stirring speed as well as the kind of stirrers such as different stirrer types and the way, how is stirred have a strong influence on the reaction.

It was observed that at higher stirring speeds the formation of caprolactam selectivity increases. It is assumed that at higher stirring speeds products more easily desorpt from the active sites of the catalyst.

The amount of catalyst used in the reaction was about 0,01 to 2 g per 10 g of ketone, preferably 0,5 to 1 g.

Usually, the reaction duration is about 2 to 24 h, preferably, the reaction is carried out at least up to 4 h to obtain maximum caprolactam yield.

Catalyst recycling is achieved by recovering the spent catalyst from the reaction mixture by filtration or decantation. The recovered catalyst is tested again for its catalytic activity in this reaction. After stopping the reaction after a certain time, the reaction mixture is cooled down to room temperature and the catalyst is filtered off from the reaction mixture.

The recovered catalyst can be washed with solvents, dried and finally calcined in an oven if necessary, after being separated from the reaction mixture. The recovered catalyst can be used again in the reaction for several runs. Alternatively or in addition, the catalyst can be regenerated via oxidative regeneration with an oxidant, e.g. oxygen.

The production of caprolactam is preferably conducted in a batchwise manner. In this batchwise manner, the reaction medium was heated to reaction temperature and in the reactor, it was charged with the required amount of cyclohexanone, ammonia, solvent and catalyst. Hydrogen peroxide is introduced continuously for 2 h by slow addition into the reaction mixture under stirring for avoiding decomposition.

It is preferred to use a glass reactor and/or a stainless steel autoclave reactor.

In general, the reactions were carried out using a mechanical stirrer; the amount of catalysts and the molar ratio of reactants were varied.

In accordance with present invention, cyclohexanone was subjected to ammoximation with a mixture of ammonia and hydrogen peroxide by using Ti-MCM-41, Al-MCM-41 and Ti-SBA-15 as catalysts for producing high yields of caprolactam.

Reaction results demonstrate that in presence of non mesoporous materials as catalysts, both higher conversion rates and selectivity of cyclohexanone to cyclohexanone oxime instead of conversion into the corresponding caprolactam were observed. This might be due to the limitation of the pore sizes of the catalysts. However, the reaction can also take place at the outer surface and at the pore mouth.

The catalytic results derived from a reaction that is carried out using 5 % TiO₂ impregnated on a SiO₂ support also have shown high cyclohexanone conversion with higher cyclohexanone oxime formation than caprolactam formation.

In order to better understand the present invention, the following illustrative examples are provided.

### Examples

In these examples cyclohexanone, cyclohexanone oxime, caprolactam and other compounds such as 2-cyclohexenylcyclohexanone isomers, were analyzed by gas-chromatography. Conversion of cyclohexanone and selectivity of the caprolactam was calculated on the basis of analytical results.

### Reaction procedure:

A 50 ml 2 necked round bottom (RB) glass flask was charged with cyclohexanone, aqueous ammonia solution (28 wt% solution), solvent ethanol (10 wt%) and catalyst amount 0.2 g. This RB flask was fitted with a reflux condenser for cooling down the reactants and products. This charged flask had been submerged in an oil bath where required temperature was adjusted. The RPM of the magnetic stirrer and the desired temperature were adjusted. The required amount of hydrogen peroxide (30 wt %) was introduced into the reactor by continuous addition for 2 h by using syringe through the septum.

Reaction conditions were as follows H₂O₂/cyclohexanone-1.32(molar ratio) and NH₃/cyclohexanone-1.94(molar ratio); solvent ethanol(10 wt%) and catalyst amount 0.2 g and reaction time was 4 h. The reaction was performed at a temperature 80 °C under ambient pressure with reaction time of 240 minutes. Afterwards, the reactor was cooled down to room temperature. Products and catalysts were separated by filtration or centrifuging method. To this separated reaction mixture, an external standard was added and samples were analyzed by gas-chromatography.

### Example 1

Ti-MCM-41 (0,2 g) was used as a catalyst and the reaction was carried out by the following method.

A 50 ml 2 necked round bottom (RB) glass flask equipped with a reflux condenser was charged with cyclohexanone (2,5 g), aqueous ammonia solution (28 wt%, 3 g), solvent ethanol (10 wt%, 0,93 g) and catalyst (0,2 g). This charged flask was submerged in an oil bath where the required temperature was adjusted. The speed of the magnetic stirrer was controlled. The required amount of hydrogen peroxide (30 wt%, 3,836 g) was introduced into the reactor by continuous addition for 2 h via a syringe.

The reaction was performed at a temperature of 80 °C under ambient pressure with a reaction time of 240 minutes. After 240 minutes of the reaction time, the reactor was cooled down to room temperature. Products and catalysts were separated by filtration or centrifugation. To this separated reaction mixture, an external standard was added and samples were taken and analyzed by gas-chromatography.

### Example 2

The reaction was carried out as in example 1, except Ti-MCM-41 is replaced by Al-MCM-41 as catalyst. The reaction was performed for 240 minutes. High cyclohexanone conversion and high selectivity to caprolactam were observed.

### Example 3

The procedures as described in example 1 were repeated with TS-1 instead of Ti-MCM-41 as catalyst. The reaction results showed that this catalyst is highly active and selective for simultaneous cyclohexanone oxime and caprolactam formation.

### Example 4

The reaction was carried out with Ti-SBA-15 following the procedure of example 1. After 240 minutes, high cyclohexanone conversion and caprolactam selectivity was observed.

### Example 5

In order to reproduce the results, the reactions were repeated several times with Al-MCM-41 catalyst under identical reaction conditions as described in example 1. After repeating the reaction five times, the results showed roughly constant conversion and selectivity on the formation of caprolactam.

### Example 6

The influence of the stirring speed in the reaction was studied by using Al-MCM-41 catalyst. Experiments were carried out in the same manner as in example 1, except changing the different stirring speeds. The results show that the caprolactam selectivity and yield increased by increasing the stirring speeds to an optimum near 900 to 1100 rpm.

### Examples 7 to 11

### Reaction procedure:

| | |
|---|---|
| NH₃: | cyclohexanone = 1.95 (molar ratio); |
| H₂O₂: | cyclohexanone = 1.35 (molar ratio) |

| | |
|---|---|
| Catalyst weight: | 0.2 g |
| Pressure: | Atmospheric |
| Solvent: | Ethanol (10 wt%) |
| Stirring speed: | 900 rpm |
| Reaction time: | 4 h |
| Temperature: | 80 °C |

**Table 1**

| Ex. | Catalysts | C.Hexanone conversion wt% | C.Hexanone oxime selectivity % | C.Lactam selectivity % | Others selectivity % |
|---|---|---|---|---|---|
| 7 | B-MFI | 6.84 | 9.06 | 34.23 | 56.71 |
| 8 | Al-ZSM-5(Si/Al-300) | 13.10 | 4.72 | 18.12 | 77.14 |
| 9 | B-Al-MFI-BA-17 (Al-0.1 g) | 7.18 | 9.93 | 42.64 | 47.43 |
| 10 | B-Al-MFI-BA-22 (Al-0.075 g) | 39.92 | 10.04 | 45.03 | 44.93 |
| 11 | B-Al-MFI-BA-19 (Al-0.05 g) | 24.36 | 9.08 | 20.54 | 70.38 |

### Borosilicate, Aluminosilicate and Boroaluminosilicate catalysts synthesis procedure

All these catalysts in examples 7 to 11 were prepared by following the method (W. Hoelderich et. al, Proceedings of the 6th International Zeolite Conference, Butterworths, Guilford, London, 1984, 545-55). 11.7 g of SiO₂ (aerosol) was dissolved in 180 g of 50 wt% aqueous hexamethylenediamines (HMD) solution and this mixture was stirred at room temperature for 3 h. Separately, in another beaker, 2.2 g of boric acid was dissolved in 9 g of HMD aqueous solution and this mixture solution was stirred at room temperature for 3 h. After 3 h stirring the mixture solution, two solutions were mixed in one beaker. Resulting to this solution mixture, 0.1 g of aluminum hydroxide was added and the whole reaction mixture was stirred at room temperature for another 2 h before transferred into stainless steel autoclave for crystallization. The complete crystallization took place at 170 °C for 5 days. After crystallization, the product was filtered off, washed with deionized water and dried at 110 °C for 12 h. Finally dried sample was calcined at 550 °C for 6 h.

In case of borosilicate, only boron source was used and in the case of aluminosilicate, only alumina source was used. But, in case of boroaluminosilicate, both sources were used simultaneously.

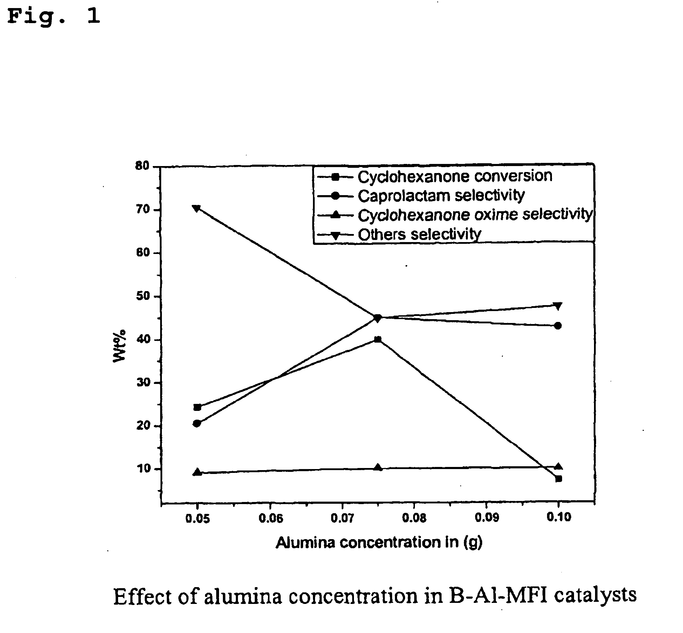

There is strong dependence up on the alumina concentration in cyclohexanone conversion and caprolactam selectivity. Fig. 1 shows that with increasing the alumina concentration from 0.05 g to 0.075 g, the cyclohexanone conversion increased from about 25 % to 40 % and caprolactam selectivity increased from about 20 % to 45 %. With further increase, the alumina concentration from 0.075 g to 0.1 g the cyclohexanone conversion and caprolactam selectivity decreased as well. However, in all cases, the constant cyclohexanone oxime selectivity observed.

### Examples 12 to 14

### Example effect of stirring speed by using Catalyst-B

The influence of the stirring speed in the reaction was studied by using Al-MCM-41 catalyst. Experiments were carried out in the same manner as in example 1, except changing the different stirring speeds. Reaction results are shown in table 2.

**Table 2**

| Ex. | Stirring speed 1/m | C.Hexanone conversion wt% | C.Lactam selectivity % | C.Oxime selectivity % |
|---|---|---|---|---|
| 12 | 500 | 17.2 | 26.0 | 4.1 |
| 13 | 900 | 18.3 | 42.7 | 3.9 |
| 14 | 1100 | 18.9 | 41.3 | 5.6 |

Results in table 2 demonstrate that the caprolactam selectivity and yield increased by increasing the stirring speeds.

## Claims

1. Method of producing a cyclic lactam of the generic formula I 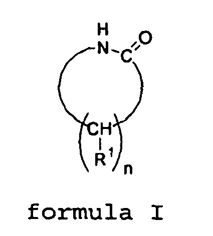 starting from cyclic ketones of the generic formula II 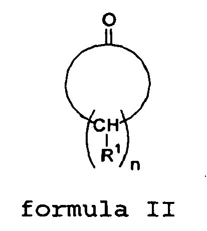 wherein
n is 2 to 20, and
R¹ independently is selected from the group consisting of hydrogen, linear or branched C₁-C₁₂-alkyl, linear or branched alkenyl with up to 12 carbon atoms, linear or branched alkinyl with up to 12 carbon atoms, aromatic residues with 6 to 18 carbon atoms, aralphatic residues with 7 to 20 carbon atoms and/or residues which contain heteroatoms,
by reacting the cyclic ketone in presence of at least one heterogeneous catalyst system with ammonia and an aqueous solution of hydrogen peroxide as at least one oxidant by conducting the reaction in one step and in liquid phase,
**characterized in that**
the catalyst system contains a material with mesoporous structure and/or zeolitic structure.

2. Method according to claim 1, **characterized in that** the heterogeneous catalyst system contains at least one material which includes transition metals and/or IIIA and/or IVA main group elements.

3. Method according to one of the preceding claims, **characterized in that** the catalyst contains a material selected from the group of the mesoporous molecular sieves.

4. Method according to the preceding claim, **characterized in that** the mesoporous molecular sieves are selected from the group consisting of materials with MCM-41-structure, MCM-48-structure, HMS-structure and/or SBA-15-structure.

5. Method according to one of the preceding claims, **characterized in that** the catalyst system contains Al, Si, Ti, Mn, Sn, V, Mo, Cu, Co, Fe, B, Ga, Ge, Nb and/or Mg and mixtures thereof.

6. Method according to one of the preceding claims, **characterized in that** the reaction is carried out at temperatures from 0 °C to 120 °C, preferably from 60 °C to 100 °C, especially preferred from 70 °C to 90 °C.

7. Method according to one of the preceding claims, **characterized in that** the reaction is carried out at pressures from 0,1 to 100 bar, preferably from 0,5 to 40 bar, especially preferred from 10 to 30 bar.

8. Method according to one of the preceding claims, **characterized in that** the at least one oxidant is used in a molar ratio to the cyclic ketone of the generic formula II from 0,5 to 3, preferably from 0,5 to 1,5, most preferably from 1 to 1,3.

9. Method according to one of the preceding claims, **characterized in that** ammonia is used in aqueous solution.

10. Method according to one of the preceding claims, **characterized in that** ammonia is used in a molar ratio to the cyclic ketone of the generic formula II from 0,5 to 3, preferably from 1 to 2,5, in particular from 1,5 to 2.

11. Method according to one of the preceding claims, **characterized in that** the at least one heterogeneous catalyst system is used in a weight ratio to the cyclic ketone of the generic formula II from 2 to 40, preferably from 4 to 12.

12. Method according to one of the preceding claims, **characterized in that** the reaction is carried out under stirring, especially at stirring speeds ranging from 500 to 1500 rpm, preferably from 500 to 1100 rpm, especially preferred from 900 to 1100 rpm.

13. Method according to one of the preceding claims, **characterized in that** the reaction is carried out in at least one solvent, being selected from the group consisting of alcohols, especially methanol, ethanol, n-propanol, i-propanol, n-butanol, s-butanol, t-butanol and/or acetone, acetonitrile, water and/or mixtures thereof.

14. Method according to the preceding claim, **characterized in that** the at least one solvent is used in a weight ratio to the cyclic ketone of the formula II from 0 to 100 weight-%, preferably from 5 to 30 weight-%.

15. Method according to one of the preceding claims, **characterized in that** the reaction is carried out over 0,5 to 24 h, preferably 2 to 12 h, most preferably 2 to 6 h.

16. Method according to one of the preceding claims, **characterized in that** the catalyst is recycled and reused after completion of the reaction.

17. Method according to the preceding claim, **characterized in that** after recycling of the catalyst a regeneration procedure of the catalyst is carried out, preferably by washing the catalyst with solvents and/or via oxidative regeneration with an oxidant, e.g. oxygen.

18. Method according to one of the preceding claims, **characterized in that** the reaction is carried out in a glass reactor and/or a stainless steel reactor.

19. Method according to one of the preceding claims, **characterized in that** the reaction is carried out in a batch reactor, autoclave, loop reactor, continuous flow fixed bed reactor, trickle bed reactor and/or moving bed reactor.

20. Method according to one of the preceding claims, **characterized in that** n is 6 or 12 and R¹ is hydrogen.

## Patentansprüche

1. Verfahren zur Herstellung eines zyklischen Lactams der allgemeinen Formel I 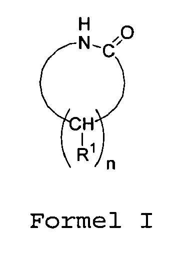 ausgehend von zyklischen Ketonen der allgemeinen Formel II 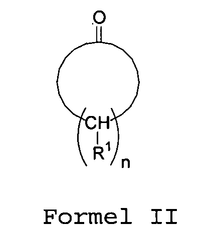 wobei
n 2 bis 20 ist, und
R¹ unabhängig ausgewählt aus der Gruppe bestehend aus Wasserstoff, linearen oder verzweigten C₁-C₁₂-Alkylresten, linearen oder verzweigten Alkenylresten mit bis zu 12 Kohlenstoffatomen, linearen oder verzweigten Alkenylresten mit bis zu 12 Kohlenstoffatomen, aromatischen Resten mit 6 bis 18 Kohlenstoffatomen, aralphatischen Resten mit 7 bis 20 Kohlenstoffatomen und/oder Resten, die Heteroatome enthalten,
durch Reaktion des zyklischen Ketons in Gegenwart zumindest eines heterogenen Katalysatorsystems mit Ammoniak und einer wässrigen Lösung von Wasserstoffperoxid als zumindest einem Oxidationsmittel unter Durchführung der Reaktionen in einem Schritt und in flüssiger Phase, **dadurch gekennzeichnet, dass** das Katalysatorsystem ein Material mit mesoporöser Struktur und/oder zeolithischer Struktur enthält.

2. Verfahren gemäß Anspruch 1, **dadurch gekennzeichnet, dass** das heterogene Katalysatorsystem zumindest ein Material enthält, das Übergangsmetalle und/oder IIIA- und/oder IVA-Hauptgruppenelemente enthält.

3. Verfahren gemäß einem der vorhergehenden Ansprüche, **dadurch gekennzeichnet, dass** der Katalysator ein Material ausgewählt aus der Gruppe der mesoporösen Molekularsiebe enthält.

4. Verfahren gemäß dem vorhergehenden Anspruch, **dadurch gekennzeichnet, dass** die mesoporösen Molekularsiebe ausgewählt sind aus der Gruppe bestehend aus Materialien mit einer MCM-41-Struktur, MCM-48-Struktur, HMS-Struktur und/oder SBA-15-Struktur.

5. Verfahren gemäß einem der vorhergehenden Ansprüche, **dadurch gekennzeichnet, dass** das Katalysatorsystem Al, Si, Ti, Mn, Sn, V, Mo, Cu, Co, Fe, B, Ga, Ge, Nb und/oder Mg und Mischungen daraus enthält.

6. Verfahren gemäß einem der vorhergehenden Ansprüche, **dadurch gekennzeichnet, dass** die Reaktion bei Temperaturen zwischen 0 °C und 120 °C, bevorzugt zwischen 60 °C und 100 °C, besonders bevorzugt zwischen 70 °C und 90 °C durchgeführt wird.

7. Verfahren gemäß einem der vorhergehenden Ansprüche, **dadurch gekennzeichnet, dass** die Reaktion bei Drücken von 0,1 bis 100 bar, bevorzugt von 0,5 bis 40 bar, besonders bevorzugt von 10 bis 30 bar ausgeführt wird.

8. Verfahren gemäß einem der vorhergehenden Ansprüche, **dadurch gekennzeichnet, dass** das zumindest eine Oxidationsmittel in einem molaren Verhältnis bezüglich des zyklischen Ketons der allgemeinen Formel II von 0,5 bis 3, bevorzugt von 0,5 bis 1,5, besonders bevorzugt von 1 bis 1,3 verwendet wird.

9. Verfahren gemäß einem der vorhergehenden Ansprüche, **dadurch gekennzeichnet, dass** Ammoniak in wässriger Lösung verwendet wird.

10. Verfahren gemäß einem der vorhergehenden Ansprüche, **dadurch gekennzeichnet, dass** Ammoniak in einem molaren Verhältnis bezüglich des zyklischen Ketons der allgemeinen Formel II von 0,5 bis 3, bevorzugt von 1 bis 2,5, insbesondere von 1,5 bis 2 verwendet wird.

11. Verfahren gemäß einem der vorhergehenden Ansprüche, **dadurch gekennzeichnet, dass** das zumindest eine heterogene Katalysatorsystem in einem Gewichtsverhältnis bezüglich des zyklischen Ketons der allgemeinen Formel II von 2 bis 40, bevorzugt von 4 bis 12 verwendet wird.

12. Verfahren gemäß einem der vorhergehenden Ansprüche, **dadurch gekennzeichnet, dass** die Reaktion unter Rühren, insbesondere bei Rührgeschwindigkeiten von 500 bis 1500 rpm, bevorzugt von 500 bis 1100 rpm, besonders bevorzugt von 900 bis 1100 rpm durchgeführt wird.

13. Verfahren gemäß einem der vorhergehenden Ansprüche, **dadurch gekennzeichnet, dass** die Reaktion in zumindest einem Lösungsmittel, ausgewählt aus der Gruppe bestehend aus Alkoholen, insbesondere Methanol, Ethanol, n-Propanol, i-Propanol, n-Butanol, s-Butanol, t-Butanol und/oder Aceton, Acetonitril, Wasser und/oder Mischungen hieraus durchgeführt wird.

14. Verfahren gemäß dem vorhergehenden Anspruch, **dadurch gekennzeichnet, dass** das zumindest eine Lösungsmittel in einem Gewichtsverhältnis bezüglich des zyklischen Ketons der allgemeinen Formel II von 0 bis 100 Gew.-%, bevorzugt von 5 bis 30 Gew.-% eingesetzt wird.

15. Verfahren gemäß einem der vorhergehenden Ansprüche, **dadurch gekennzeichnet, dass** die Reaktion über 0,5 bis 24 h, bevorzugt 2 bis 12 h, besonders bevorzugt 2 bis 6 h ausgeführt wird.

16. Verfahren gemäß einem der vorhergehenden Ansprüche, **dadurch gekennzeichnet, dass** der Katalysator nach Abschluss der Reaktion recycelt und wiederverwendet wird.

17. Verfahren gemäß dem vorhergehenden Anspruch, **dadurch gekennzeichnet, dass** nach Recycling des Katalysators ein Regenerationsverfahren des Katalysators durchgeführt wird, bevorzugt durch Waschen des Katalysators mit Lösungsmitteln und/oder durch oxidative Regeneration mit einem Oxidationsmittel, z.B. Sauerstoff.

18. Verfahren gemäß einem der vorhergehenden Ansprüche, **dadurch gekennzeichnet, dass** die Reaktion in einem Glasreaktor und/oder einem Reaktor aus rostfreiem Stahl ausgeführt wird.

19. Verfahren gemäß einem der vorhergehenden Ansprüche, **dadurch gekennzeichnet, dass** die Reaktion in einem Batch-Reaktor, Autoklaven, Schleifenreaktor, in einem kontinuierlichen Fließbettreaktor, in einem Rieselbettreaktor und/oder einem Wanderbettreaktor durchgeführt wird.

20. Verfahren gemäß einem der vorhergehenden Ansprüche, **dadurch gekennzeichnet, dass** n 6 bis 12 und R¹ Wasserstoff ist.

## Revendications

1. Procédé de production d'un lactame cyclique répondant à la formule générique I : 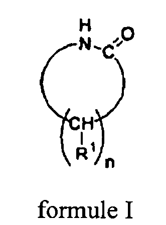 en partant de cétones cycliques répondant à la formule générique II : 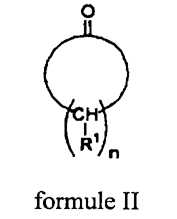 dans laquelle
n vaut 2 à 20, et
R¹ est choisi indépendamment parmi le groupe constitué de l'hydrogène, d'un alkyle à C₁ à C₁₂ linéaire ou ramifié, d'un alcényle linéaire ou ramifié contenant jusqu'à 12 atomes de carbone, d'un alcynyle linéaire ou ramifié contenant jusqu'à 12 atomes de carbone, de résidus aromatiques ayant 6 à 18 atomes de carbone, de résidus araliphatiques ayant 7 à 20 atomes de carbone et/ou de résidus qui contiennent des hétéroatomes,
en faisant réagir la cétone cyclique, en présence d'au moins un système de catalyseur hétérogène, avec de l'ammoniac et avec une solution aqueuse de peroxyde d'hydrogène comme au moins un oxydant, la réaction étant effectuée en une seule étape et en phase liquide,
**caractérisé en ce que**
le système de catalyseur contient un matériau ayant une structure mésoporeuse et/ou une structure zéolitique.

2. Procédé selon la revendication 1, **caractérisé en ce que** le système de catalyseur hétérogène contient au moins un matériau qui comprend des métaux de transition et/ou des éléments du groupe principal IIIA et/ou IVA.

3. Procédé selon l'une des revendications précédentes, **caractérisé en ce que** le catalyseur contient un matériau choisi parmi le groupe des tamis moléculaires mésoporeux.

4. Procédé selon la revendication précédente, **caractérisé en ce que** les tamis moléculaires mésoporeux sont choisi parmi le groupe constitué de matériaux ayant une structure de type MCM-41, une structure de type MCM-48, une structure de type HMS et/ou une structure de type SBA-15.

5. Procédé selon l'une des revendications précédentes, **caractérisé en ce que** le système de catalyseur contient un élément de type Al, Si, Ti, Mn, Sn, V, Mo, Cu, Co, Fe, B, Ga, Ge, Nb et/ou Mg et des mélanges de ceux-ci.

6. Procédé selon l'une des revendications précédentes, **caractérisé en ce que** la réaction est effectuée à des températures dans la plage de 0 °C à 120 °C, de préférence de 60 °C à 100 °C, mieux encore de 70 °C à 90 °C.

7. Procédé selon l'une des revendications précédentes, **caractérisé en ce que** la réaction est effectuée à des pressions dans la plage de 0,1 à 100 bars, de préférence de 0,5 à 40 bars, mieux encore de 10 à 30 bars.

8. Procédé selon l'une des revendications précédentes, **caractérisé en ce que** le au moins un oxydant est utilisé dans un rapport molaire vis-à-vis de la cétone cyclique de formule générique II, de 0,5 à 3, de préférence de 0,5 à 1,5, de la manière la plus préférée de 1 à 1,3.

9. Procédé selon l'une des revendications précédentes, **caractérisé en ce que** l'ammoniac est utilisé en solution aqueuse.

10. Procédé selon l'une des revendications précédentes, **caractérisé en ce que** l'ammoniac est utilisé dans un rapport molaire vis-à-vis de la cétone cyclique de formule générique II, de 0,5 à 3, de préférence de 1 à 2,5, en particulier de 1,5 à 2.

11. Procédé selon l'une des revendications précédentes, **caractérisé en ce que** le au moins un système de catalyseur hétérogène est utilisé dans un rapport pondéral vis-à-vis de la cétone cyclique de formule générique II, de 2 à 40, de préférence de 4 à 12.

12. Procédé selon l'une des revendications précédentes, **caractérisé en ce que** la réaction est effectuée sous agitation, en particulier, à des vitesses d'agitation dans la plage de 500 à 1500 tr/min, de préférence de 500 à 1100 tr/min, mieux encore de 900 à 1100 tr/min.

13. Procédé selon l'une des revendications précédentes, **caractérisé en ce que** la réaction est effectuée dans au moins un solvant, lequel est sélectionné dans le groupe constitué des alcools, en particulier, du méthanol, de l'éthanol, du n-propanol, de l'i-propanol, du n-butanol, du s-butanol, du t-butanol et/ou de l'acétone, de l'acétonitrile, de l'eau et/ des mélanges de ceux-ci.

14. Procédé selon la revendication précédente, **caractérisé en ce que** le au moins un solvant est utilisé dans un rapport pondéral vis-à-vis de la cétone cyclique de formule II, de 0 à 100 % en poids, de préférence de 5 à 30 % en poids.

15. Procédé selon l'une des revendications précédentes, **caractérisé en ce que** la réaction est effectuée sur une période de 0,5 à 24 h, de préférence, de 2 à 12 h, mieux encore de 2 à 6 h.

16. Procédé selon l'une des revendications précédentes, **caractérisé en ce que** le catalyseur est recyclé et réutilisé après que la réaction est achevée.

17. Procédé selon l'une des revendications précédentes, **caractérisé en ce que**, après avoir recyclé le catalyseur, une procédure de régénération du catalyseur est effectuée, de préférence, en lavant le catalyseur avec des solvants et/ou par une régénération oxydante en utilisant un oxydant, par exemple, de l'oxygène.

18. Procédé selon l'une des revendications précédentes, **caractérisé en ce que** la réaction est effectuée dans un réacteur en verre et/ou dans un réacteur en acier inoxydable.

19. Procédé selon l'une des revendications précédentes, **caractérisé en ce que** la réaction est effectuée dans un réacteur discontinu, dans un autoclave, dans un réacteur à boucle, dans un réacteur à lit fixe en flux continu, dans un réacteur à lit ruisselant et/ou dans un réacteur à lit mobile.

20. Procédé selon l'une des revendications précédentes, **caractérisé en ce que** n vaut 6 ou 12 et **en ce que** R¹ est de l'hydrogène.
